# EUROPEAN PATENT APPLICATION

(11) **EP 1 226 824 A1**
(43) Date of publication of application: **31.07.2002**
(21) Application number: 01300601.0
(22) Date of filing: 24.01.2001
(51) Int. Cl.: A61K 31/454, A61P 35/00, A61P 1/16

(54) **Use of thalidomide for the treatment of hepatocellular carcinoma**

(71) Applicant: TTY Biopharm Company Limited, Taipei, Taiwan (TW)
(72) Inventor: Huang, Chun-Ying, Taipei (TW); Whang-Peng, Jia-Kang, Taipei (TW); Chen, Li-Tzong, Kaohsiung City (TW); Liu, Tsang-Wu, Taipei (TW); Chang, Jang-Yang, Taipei (TW); Hsu, Ming-Chu, Taipei (TW)
(74) Representative: Woods, Geoffrey Corlett

(57) **Abstract**

The invention discloses the use of thalidomide in the manufacture of a medicament for the treatment of hepatocellular carcinoma.

## Description

### BACKGROUND OF THE INVENTION

Thalidomide was first synthesized in 1953, and it was widely used as a sedative and for the prevention of vomiting during pregnancy. In 1963, it was found that women who took thalidomide in the first trimester of pregnancy would deliver terata, such as phocomelia. Therefore, thalidomide was prohibited in Europe and the USA.

In view of studies in recent years, thalidomide has the efficacy on adjustment of the immune system which may treat immune system related diseases. For instance, Arch Dermatol. 1993, vol. 129, p. 1548-1550 described the use of thalidomide in the treatment of cutaneous lupus erythematosus; the Journal of Rheumatology, 1989, 16, p. 159-163 described the use of thalidomide in the treatment of refractory rheumatoid arthritis; Arch Dermatol. 1990, vol. 126, p. 923-927 described the use of thalidomide in the treatment of Behcet's syndrome; Journal of Pediatr. Gastroenerol. Nurt. 1999, vol. 28, p. 214-216 described the use of thalidomide in the treatment of Cornh's disease; and Journal of Rheumatology, 1998, vol. 25, p. 964-969 described the use of thalidomide in the treatment of rheumatoid arthritis. In addition, US Patent Nos. 5,593,990 and 5,629,327 disclose that thalidomide could effectively inhibit angiogenesis; US Patent No. 5,654,312 discloses the methods of treatment for inflammatory and autoimmune dermatoses. In addition, the Journal of Infectious Diseases, 1993, 168, p. 408-414 taught that thalidomide could effectively inhibit tumor necrotic factor-alpha (TNF-I). Anti-Cancer Drugs, 1996, 7, p. 339-343 demonstrated that thalidomide could effectively inhibit basic fibroblast growth factor-induced angiogenesis. Thalidomide is widely applied in the clinical treatment of malignant tumors which are highly vascular and cannot be effectively treated by chemical therapy. For instance, US Patent No. 5,696,092 discloses the use of thalidomide in the inhibition of metastases of cancers of epithelial cell origin, especially human prostate cancers. Among the above prior art references, none of the references or patents teaches that thalidomide could be specifically used in the treatment of hepatocellular carcinoma.

Up to the present time, there are not any drugs that can effectively treat hepatocellular carcinoma. Patients with metastatic hepatocellular carcinoma or hepatocellular carcinoma, where local treatment has failed, normally survive for only three to four months. Metastatic hepatocellular carcinoma or hepatocellular carcinoma, where local treatment has failed, is mainly subjected to systemic therapy. The use of Doxorubicin, a high dosage of Tamoxifen in combination Doxorubicin or EA-PFL (etopoxide, adrimycin, cisplatin, fluorouracil and leucovorin), is an effective example. The remission rate of those drugs can achieve levels between 15 and 30%. However, because the patients of hepatocellular carcinoma usually develop complication of liver cirrhosis and other complications (such as Ieukopenia, thrombopenia or liver function impairment), they cannot be subject to systemic chemotherapy.

### DESCRIPTION OF THE DRAWINGS

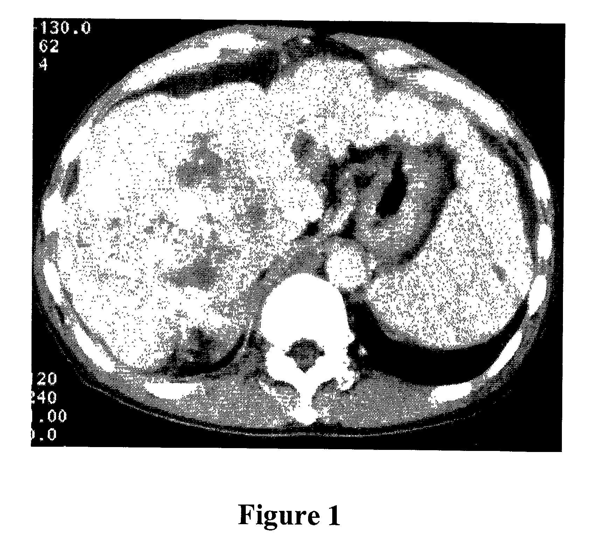

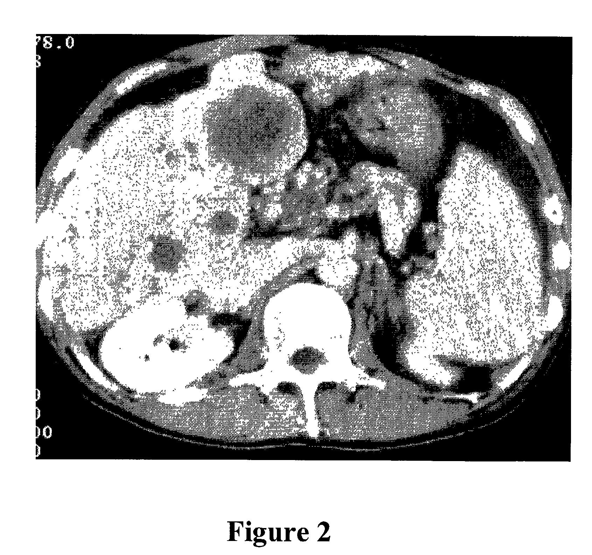

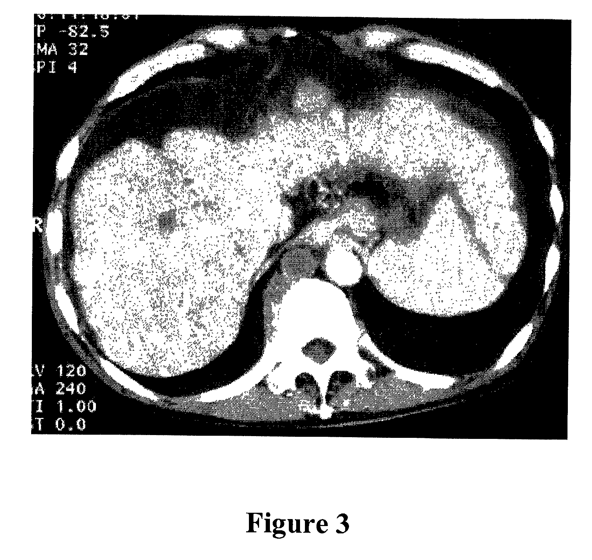

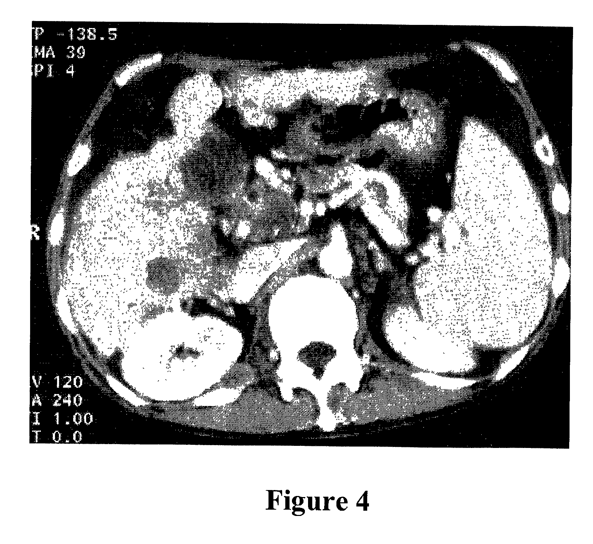

Figures 1-4 show computerized abdominal tomography of a patient, before and after, treatment with thalidomide. Figure 1 and 2: before the treatment with thalidomide, the computerized abdominal tomography scan shows that the left and right hepatic lobes of the patient were infiltrated with diffused hepatocellular carcinoma. The depositing of Lipiodol on the liver lobes after arterial embolization is shown in Figures 1 and 2. Figure 2 also shows a 5 cm × 5 cm massive type index lesion at the left hepatic lobes. The serum level of alpha-fetoprotein in the patient is 4335 µg/ml. Figures 3 and 4: after treatment with thalidomide, the computerized abdominal tomography scan shows that most diffused hepatocellular carcinoma, which infiltrated the left and right hepatic lobes of the patient, disappear. The massive type index lesion at the left hepatic lobe shown in Figure 3 has been reduced to the size of 3 cm × 3 cm. The serum level of alpha-fetoprotein in the patient is 1501 µg/ml. In addition, the scan show the occurrence of ascitic fluid. After the detection by abdominal paracentesis, it is proved that the occurrence of ascitic fluid was caused by spontaneous bacterial peritonitis, and hepatocellular carcinoma does not exist.

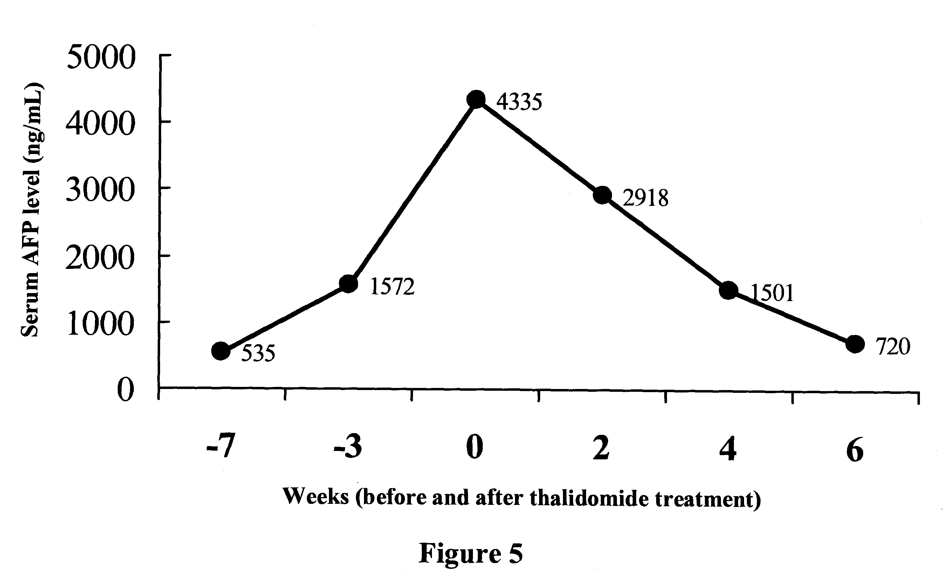

Figures 5-7 show the variation of the serum level of alpha-fetoprotein in three individual patient before and after the treatment with thalidomide.

### SUMMARY OF THE INVENTION

An object of the subject invention is to provide a pharmaceutical composition for use in the treatment of hepatocellular carcinoma.

Another object of the subject invention is to provide a pharmaceutical composition for use in the treatment of metastatic hepatocellular carcinoma or hepatocellular carcinoma, where local treatment has failed, which comprises thalidomide and a pharmaceutically acceptable carrier.

Another object of the subject invention is to provide a pharmaceutical composition used as adjuvant treatment for patients of hepatocellular carcinoma who have failed to local treatment, such as percutaneous ethanol injection, operation, transcatheter arterial chemoembolization (TACE) or cryotherapy.

### DETAILED DESCRIPTION OF THE INVENTION

The subject invention utilizes thalidomide to treat metastatic hepatocellular carcinoma and hepatocellular carcinoma, where local treatment has failed. The invention found that thalidomide has excellent effects concerning the treatment of such carcinoma which are difficult to treat. This includes the significant and rapid decrease of the serum level of alpha-fetoprotein, the reduction of tumors and the relief of symptoms for patients, without significant side effects, such as arrest of bone marrow or hepatotoxicity.

The chemical nomenclature of thalidomide used in the subject invention is 2-(2,6-dioxo-3-piperidinyl)-1H-isoindolle-1,3(2H)-dione, which is a white crystal powder; odorless; mp 269-271 °C; sparingly soluble in water, methanol, ethanol or acetone. The chemical structure of thalidomide is as follows:

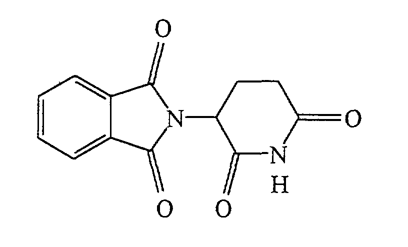

The term "pharmaceutically effective amount" used in the pharmaceutical composition of the subject invention is directed to the administered amount to mammals that need such treatment in order to proceed with the above-mentioned treatment. The pharmaceutically effective amount depends on the individual, the disease to be treated, the body weight and age of the individual, the level of the disease or the administration route. This can be determined by persons skilled in the art. The pharmaceutically effective amount of thalidomide used in the subject invention is 30 to 1200 mg for an adult for a daily dose of oral administration, preferably 50 to 800 mg and more preferably 100 to 500 mg.

The pharmaceutical composition of the subject invention can be used in combination with other hepatocellular carcinoma treating drugs, such as anticancer chemotherapeutic drugs, hormones, biological response modifier(s), other angiogenesis inhibitors; or in combination with immunotherapy or gene therapy.

The pharmaceutical composition of the subject invention can be administered by different routes, comprising oral, rectal, topical subcutaneous, intravenous, intramuscular and nasal administration. The compound is effective in both injective formulation or oral formulation.

The pharmaceutical composition of the subject invention can be formulated by use of conventional techniques as discrete dosage forms, such as capsules, cachets, tablets, granules or pills; a solution or a suspension in an aqueous liquid or a non-aqueous liquid; or as an oil-in-water liquid emulsion or a water-in-oil emulsion and as a bolus; together with suitable pharmaceutically acceptable carrier. For instance, a table may be made by compression or molding, optionally with one or more excipient or carrier ingredients. Compressed tables may be prepared by compressing, in a suitable machine, thalidomide in a free-flowing form such as a powder or granules, mixed with a binder, flavoring agent, solubilizer, lubricant, inert diluent, preservative, surface active or dispersing agent. The table may be optionally coated or formulated so as to provide a controlled release of thalidomide.

The therapeutic efficacy of the pharmaceutical composition of the subject invention comprising thalidomide on the treatment of hepatocellular carcinoma has been supported by clinical observation as illustrated in the following examples.

### Example

### Example 1

Capsules each containing 50 mg of thalidomide were made as follow: thalidomide 50 mg, lactose 50 mg, corn starch 18 mg, and Avicel 65 mg, were blended, passed through a No. 45 mesh sieve, and filled into hard gelatin capsules.

### Example 2

A 44 year-old male patient weighted 55 kg with medical history of hepatitis C was diagnosed with hepatocellular carcinoma in December 1998 and treated with transcatheter arterial chemoembolization. He was treated with transcatheter arterial chemoembolization again in March and June 1999. According to the computerized abdominal tomography and gastrointestinal track barium enema, hepatocellular carcinoma invasion of the right colon and duodenum was doubted. The patient was treated with radiation on the right liver lobe during July to September 1999. After one-month of treatment, the serum level of alpha-fetoprotein in the patient increased from 105µ g/ml, before treatment, to 535 µg/ml. The follow-up magnetic resonance imaging (MRI) revealed that the hepatocellular carcinoma of the patient exacerbated and was complicated with tumor thrombosis of a portal vein. The patient was treated with a forth transcatheter arterial chemoembolization. The serum level of alpha-fetoprotein in the patient was increased to 1572 µg/ml.

In November 1999, the follow-up computerized abdominal tomography scan showed that the two hepatic lobes of the patient had wide hepatocellular carcinoma infiltration (as shown in Figs. 1(a) and 1(b)), esophageal and gastric varices, tumor thrombosis of a portal vein and the main portal vein in the liver. The serum level of alpha-fetoprotein in the patient was up to 4335 µg/ml. The liver function exacerbated that the total bilirubin was 9.2 mg/ml, GOT/GPT was 253/115 IU and alkaline phosphase (ALP) was 239 unit/l. As the liver function of the patient was significantly exacerbated, he was not suitable to take transcatheter arterial embolization therapy. A capsule containing 100 mg of thalidomide was orally administered to the patient twice daily during the thalidomide treatment. After two weeks of treatment, right upper quadrant tenderness of the patient was significantly relieved. After four weeks, the serum level of alpha-fetoprotein in the patient was decreased to 1501 µg/ml, total bilirubin was 10.2 mg/ml, GOT/GPT was 184/102 IU and alkaline phosphase was 233 unit/l. Meanwhile, the follow-up MRI showed that the hepatocellular carcinoma of the two liver lobes significantly remitted (as shown Figures 3 and 4). However, ascitic fluid was found. The abdominal paracentesis evidenced that ascitic fluid was caused by spontaneous bacterial peritonitis. The hepatocellular carcinoma did not exist. The patient was then administered with antibiotics for the treatment of spontaneous bacterial peritonitis. The patient was still treated with thalidomide to the present. Figure 5 shows the variation of the serum level of alpha-fetoprotein in the patient. After treatment with thalidomide, the serum level of alpha-fetoprotein significantly decreased.

### Example 3

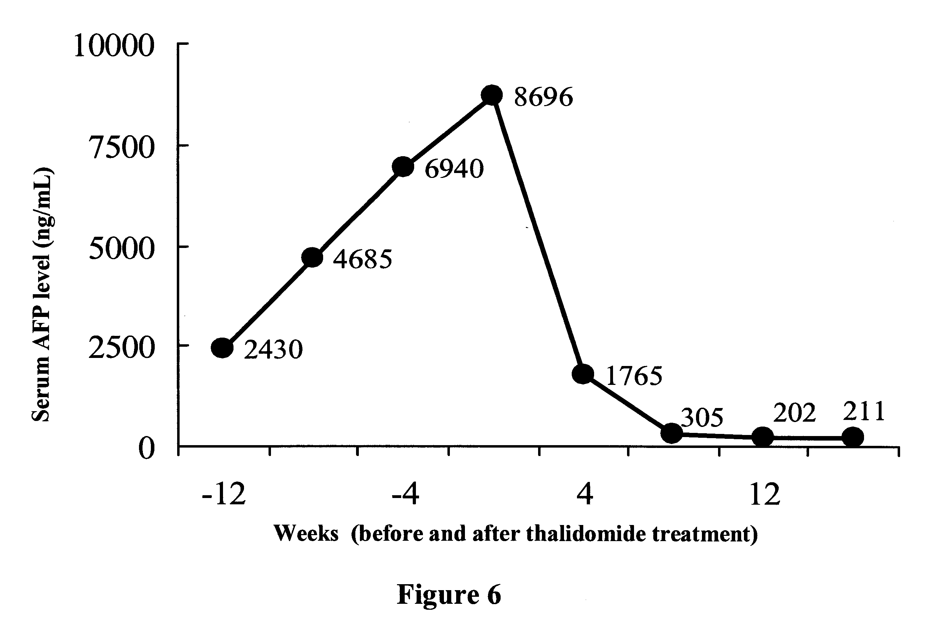

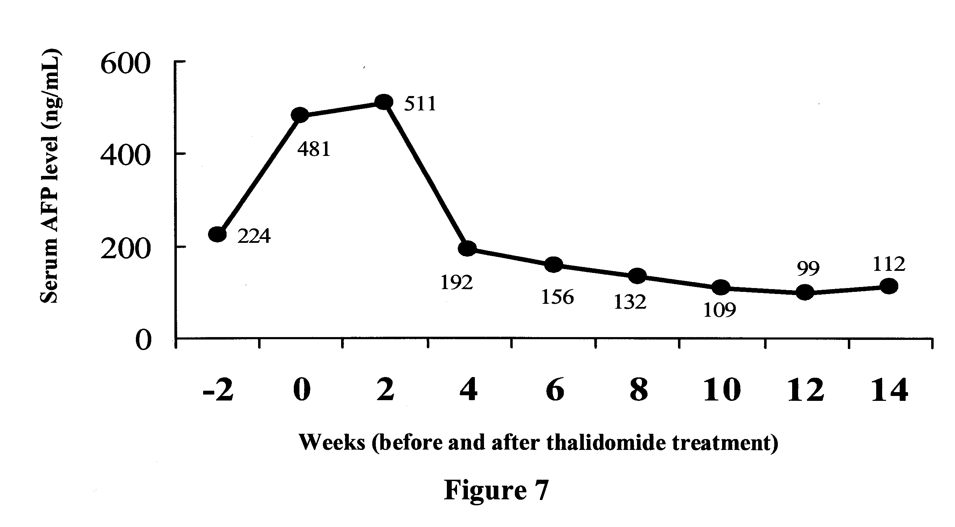

Two patients with metastatic and locally advanced hepatocellular carcinoma who were unable to have or had failed to local treatments were subjected to thalidomide treatment. Thalidomide was administered 100 mg twice daily. They were subjected to serum alpha-fetoprotein test every 2-4 weeks and computed tomography or magnetic resonance image examination every 4-8 weeks. As shown in Figures 6 and 7, serum alpha-fetoprotein level in the two patients was significant reduced by after thalidomide treatment.

## Claims

1. Use of thalidomide in the manufacture of a medicament for the treatment of hepatocellular carcinoma.

2. Use according to claim 1 wherein the medicament is adapted for the administration of 30 to 1200 mg of thalidomide.

3. Use according to claim 2, wherein the medicament is adapted for the administration of 50 to 800 mg of the lidomide.

4. Use according to any one of the preceding claims wherein the hepatocellular carcinoma is metastatic hepatocellular carcinoma of a patient who has failed to respond to local treatment.

5. Use according to any one of the preceding claims wherein the medicament is administered in combination with an additional drug for treating hepatocellular carcinoma, which additional drug is an anticancer chemotherapeutic drug, hormone, biological response modifier or other angiogenesis inhibitor.

6. Use according to any one of the preceding claims wherein the medicament is administered in combination with immunotherapy or gene therapy.

7. Use according to any one of claims 1 to 4 in which the medicament is an adjuvant treating agent in the treatment of hepatocellular carcinoma.

8. Use according to claim 7, wherein the treatment of hepatocellular carcinoma is percutaneous ethanol injection, operation, transcatheter arterial chemoembolization or cryotherapy.
